# EUROPÄISCHE PATENTSCHRIFT

(11) **EP 0 532 905 B1**
(45) Veröffentlichungstag und Bekanntmachung des Hinweises auf die Patenterteilung: **07.05.1997**
(21) Anmeldenummer: 92113722.0
(22) Anmeldetag: 12.08.1992
(51) Int. Cl.: C07C 29/76, C07C 31/20, C07C 29/80

(54) **Verfahren zur Trennung eines Flüssigkeitsgemisches**
Process for the separation of a liquid mixture
Procédé de séparation d'un mélange liquide

(30) Priorität: 14.09.1991 DE 4130661
(43) Veröffentlichungstag der Anmeldung: 24.03.1993
(73) Patentinhaber: Jehle, Walter, Dipl.-Ing., 88263 Horgenzell (DE); Schäffner, Guido, Dipl.-Ing., 88662 Überlingen (DE)
(72) Erfinder: Jehle, Walter, Dipl.-Ing., 88263 Horgenzell (DE); Schäffner, Guido, Dipl.-Ing., 88662 Überlingen (DE)

(56) Entgegenhaltungen:
- EP-A- 0 324 915
- US-A- 4 952 318
- CHEMICAL ABSTRACTS, Bd. 113, Nr. 26, 24. Dezember 1990, Columbus, Ohio, US; Zusammenfassung Nr. 237169b, Seite 353, H. SHIMIZU et al: "Recovery of antifreeze from snow removal effluents"; & JP-A-2 131 191
- CHEMICAL ABSTRACTS, Bd. 112, Nr. 6, 5. Februar 1990, Columbus, Ohio, US; Zusammenfassung Nr. 38591n, C. R. BARTELS et al: "Plant evaluation of pervaporation process", S. 160, Spalte 1
- CHEMICAL ABSTRACTS, Bd. 101, Nr. 12, 17. September 1984, Columbus, Ohio, US; Zusammenfassung Nr. 93235, T. OSADA et al: "Reverse osmotic concentration of aqueous 1, 2-ethanediol solutions", Seite 122, Spalte 1
- CHEMICAL ABSTRACTS, Bd. 111, Nr. 20, 13. November 1989, Columbus, Ohio, US; Zusammenfassung Nr. 177607q, G. A. SHUB et al: "Utilization of used antifreeze", Seite 215

## Beschreibung

Die Erfindung betrifft ein Verfahren zur Trennung eines Gemischs aus einer organischen Flüssigkeit und Wasser.

In der Bundesrepublik fallen pro Jahr mehrere 10 000 Tonnen verbrauchter Glykollösungen (Konzentration ca. 20 %) aus den Kühlern von Automobilen an. Die gebrauchten Kühlerinhalte dürfen in Zukunft nicht mehr den Kläranlagen zugeführt werden, sondern müssen als Sondermüll behandelt werden. Es besteht somit ein starker Bedarf nach einer kostengünstigen Möglichkeit zur Entsorgung bzw. Wiederverwendung der Stoffe.
Eine rein destillatives Verfahren zur Abtrennung des Glykols besitzt folgende Nachteile:
- Aufgrund verschiedener Inhaltstoffe besteht die Gefahr der Bildung von Nitrosaminen bei Temperaturen über 80 °C. Diese könnte nur durch eine aufwendige vollständige Entfernung von Nitrit und Nitrat vermieden werden.
- Zur Erzielung einer hohen Reinheit des Destillats ist ein hohes Rückflußverhältnis erforderlich, was nur mit einem hohen Energieverbrauch erreichbar ist.
- Das bei der Destillation entstehende Sumpfprodukt müßte gesondert entsorgt werden.
Eine Aufarbeitung ausschließlich nach dem Verfahren der Pervaporation ist aus wirtschaftlicher Sicht nicht sinnvoll, da die erforderliche Membranfläche sehr groß und entsprechend teuer wäre.

Der Erfindung liegt die Aufgabe zugrunde, ein wirtschaftliches Verfahren zur Trennung eines Gemischs aus Wasser und einer organischen Flüssigkeit, insbesondere Glykol, zu schaffen, wobei das abgetrennte Wasser mit einer solch hohen Reinheit anfällt, daß ein Wiedereinsatz oder ein unbeschränktes Einleiten möglich ist.
Diese Aufgabe wird mit einem Verfahren nach Anspruch 1 gelöst. Ausgestaltungen der Erfindung sind Gegenstände von Unteransprüchen.

Das erfindungsgemäße Verfahren stelle eine Verfahrenskombination aus Destillation, Pervaporation und Umkehrosmose dar. Es ist nicht auf die Abtrennung von Glykol aus Wasser beschränkt. Allgemein können damit Flüssigkeitsgemische aus Wasser und einer organischen Flüssigkeit wie z.B.
- Carbonsäuren, darunter z.B. Essigsäure, Propionsäure;
- aromatische Amine, darunter z.B. Anilin;
- Phenol
- Glycerin
getrennt werden.
Voraussetzung für die Anwendung des erfindungsgemäßen Verfahrens ist, daß solche organischen Flüssigkeiten verwendet werden, die von Wasser mit jedem der drei Einzelverfahren Destillation, selektive H₂O-Pervaporation und Umkehrosmose getrennt werden können. Die bestimmende Parameter für die Ausführbarkeit einer Destillation ist die Siedetemperatur der beiden zu trennenden Stoffe. Dagegen ist für die Ausführbarkeit einer Umkehrosmose die Molmasse von großer Bedeutung.
Das erfindungsgemäße Verfahren ist für solche organischen Flüssigkeiten angewendbar, deren Siedetemperaturen bei dem jeweiligen Druck größer als die des Wassers ist, und deren Molmasse über 40 g/mol liegt.
Die Entwicklung von geeigneten Membranen zur Ausführung einer H₂O-selektiven Pervaporation hat heutzutage einen so hohen Stand erreicht, daß für jede organische Flüssigkeit eine geeignete Membran vorhanden ist.

Das Verfahren ermöglich, die organische Flüssigkeit, die bevorzugt in einem Konzentrationsbereich von 5 - 70 % vorliegt, auf eine Konzentration von mindestens 90 %, bevorzugt ≥ 95 % anzureichern. Gleichzeitig liegt das entmischte Wasser mit einer Reinheit von mindestens 99,9 % vor und ist damit wiederverwendbar und unbeschränkt einleitfähig.

Erfindungsgemäß erfolgt zunächst eine Destillation des Gemischs. Bevorzugte Prozeßparameter sind dabei:
- Temperatur:: 70 °C - 160 °C
- Druck:: 100 mbar - 1000 mbar.
Die Destillation kann also sowohl unter Normaldruck als auch unter vermindertem Druck ausgeführt werden (Vakuumdestillation).
In nächsten Schritt erfolgt die Aufkonzentration der organischen Flüssigkeit durch Anwendung einer Pervaporation unter Verwendung wasserselektiver Membranen auf das Sumpfprodukt aus der Destillation. Bevorzugte Prozeßparameter sind hierbei:
- Temperatur:: 60 °C - 95 °C
- permeatseitiger Druck:: 20 - 150 mbar.
Die Aufbereitung des Wassers aus dem Destillat erfolgt durch eine Umkehrosmose. Das dabei anfallende Retentat wird in den Zulauf der Destillation zurückgeführt.
Bevorzugte Prozeßparameter bei der Umkehrosmose sind:
- Temperatur:: 15 °C - 35 °C
- Druck:: 20 - 70 bar.
Die abgetrennte organische Flüssigkeit liegt im Permeat aus der Pervaporation mit einer Konzentration vom mindestens 90 %, bevorzugt ≥ 95 % vor. Das abgetrennte Wasser liegt als Permeat aus der Umkehrosmose und als Permeat aus der Pervaporation vor.
Vorteilhaft kann das Permeat aus der Pervaporation zur Erhöhung der Reinheit des darin enthaltenen Wassers in den Zufluß der Umkehrosmose geleitet werden.

Durch Aktivkohle-Filter wird das abgetrennt Wasser weiter aufbereitet.

Das erfindungsgemäße Verfahren ist sowohl in technischer Hinsicht (als Produkte entstehen ein hochwertiges Konzentrat sowie wiederverwendbares, hochreines Wasser) als auch in wirtschaftlicher Hinsicht (geringer Energiebedarf, geringer Investitionsbedarf) besonders vorteilhaft.
Insbesondere kann sein Aufbau den jeweils herrschenden wirtschaftlich/technischen Rahmenbedingungen angepaßt und optimiert werden: Sollte sich z.B. in Zukunft die Pervaporation als besonders kostengünstig erweisen (bessere, billigere Membrane als bisher), so ist es möglich, die Pervaporation innerhalb des Gesamtverfahrens auszubauen und für die Destillation weniger Aufwand zu treiben. Genauso ist es möglich, falls die Umkehrosmose sich zu einem wesentlich effektiveren und wirtschaftlicheren Verfahren fortentwickelt, mehr Aufwand für die Umkehrosmose zu Betreiben und den Aufwand in der Destillation und der Pervaporation zu verringern, d.h. die Anforderungen an das Destillat bzw. an das Permeat zu reduzieren.

Die Erfindung wird anhand einer Fig. näher erläutert. Die zeigt eine schematische Darstellung des erfindungsgemäßen Verfahrens am Beispiel der Glykol-Rückgewinnung aus gebrauchten Kühlerinhalten.
Nachdem das gebrauchte Kühlerwasser von Feststoffen und unerwünschten Ionen befreit ist, wird das Flüssigkeitsgemisch Wasser/Glykol, das üblicherweise mit einer Glykolkonzentration von 20 - 30 % vorliegt, einer Vakuumdestillation unterzogen. Bedingt durch den guten Trennfaktor (a = 50) infolge des großen Differenz der Siedetemperaturen fällt das Wasser im Destillat mindestens mit einer Reinheit von 99 % an. Das Sumpfprodukt wird mit einer Konzentration vom mindestens 60 % Glykol abgezogen. Bei einem Vakuum von 100 mbar werden vorteilhaft Temperaturen von 70 - 80 °C erreicht.
In der nächsten Stufe wird mit einer Pervaporationsanlage mit Hilfe von wasserselektiven Membranen das Glykol aufkonzentriert. Es fällt im Retentat mit einer Reinheit von mindestens 95 % an. Je nach Konzentration des Wassers im Permeat (erreichbar ist eine Reinheit des Wasser von mindestens 99,95 %) und Art der Verwendung wird es eventuell einer zusätzlichen Reinigung unterzogen.
Die Aufbereitung des Wassers aus dem Destillat und gegebenenfalls aus dem Permeat der Pervaporation wird in einer Umkehrosmose durchgeführt.
Das hier anfallende Retentat enthält eine etwa um den Faktor 10 höhere Glykolkonzentration gegenüber dem Zufluß (Feed). Das Retentat wird zum Feed der Destillation zurückgeführt.

Das gewonnene Wasser wird falls erforderlich anschließend über ein Aktivkohlefilter geleitet.

Soll beispielsweise ein Glykol-Wassergemisch mit 20 % Glykol-Anteil getrennt werden, so ergeben sich rechnerisch, ausgehend von einem Massenstrom von 50 kg/h, die folgenden Massenströme und Konzentrationen:

| | |
|---|---|
| Zufluß für die Destillation | 53,94 kg/h mit 18,59 % Glykol |
| Sumpfprodukt = Zufluß für Pervaporation | 14,28 kg/h mit 70,00 % Glykol |
| Retentat aus Pervaporation | 10,50 kg/h mit 95,00 % Glykol |
| Permeat aus Pervaporation | 3,77 kg/h mit 0,50 % Glykol |
| Destillat | 39,66 kg/h mit 0,50 % Glykol |

| | |
|---|---|
| Zufluß für Umkehrosmose = Destillat + Permeat aus Pervaporation | 43,44 kg/h mit 0,50 % Glykol |
| Retentat aus Umkehrosmose | 3,95 kg/h mit 5,00 % Glykol |
| Permeat aus Umkehrosmose | 39,49 kg/h mit 0,05 % Glykol |

Bei dieser Berechnung wurden für den Trennfaktor der Destillation und für den Rückhaltegrad bei der Umkehrosmose und Pervaporation jeweils Werte angesetzt, die mit kommerziell erhältlicher Ausstattung erreichbar sind.

In Laborversuchen wurden bei der Durchführung einer einstufigen Umkehrosmose folgende Ergebnisse erzielt:
a) verwendete Membran: Membran 810 der Fa. Torray

| Feedkonz. [%] | Fluß [kg/(m2*h)] | Permeatkonz. [%] | Rückhaltegrad |
|---|---|---|---|
| 0,1 | 59,5 | 0.013 | 87,0 |
| 0,5 | 51,9 | 0,052 | 89,8 |
| 1,0 | 45,6 | 0,155 | 88,6 |
| 5,0 | 20,8 | 0,880 | 82,4 |

b) verwendete Membran: Membran Desal 3B der Fa Desalination

| Feedkonz. [%] | Fluß [kg/(m2*h)] | Permeatkonz. [%] | Rückhaltegrad |
|---|---|---|---|
| 0,1 | 33,3 | 0,016 | 85,0 |
| 0,5 | 35,1 | 0,053 | 89,5 |
| 1,0 | 21,1 | 0,126 | 87,4 |
| 5,0 | 12.4 | 1,450 | 71,0 |

Die Versuche zu a) und b) wurden jeweils bei einem Druck von 40 bar und einer Temperatur von 20 °C mit einem Glykol/Wassergemisch durchgeführt.

In Laborversuchen wurden bei der Durchführung einer wasser-selektiven Pervaporation folgende Ergebnisse erzielt:

| | |
|---|---|
| Feedkonzentration: | 90,0% |
| Fluß: | 1,2 kg/(m2*h) |
| Permeatkonzentration: | 0,6 % |
| Trennfaktor: | 1546 |

Die Versuche wurden mit einem Glykol/Wassergemisch bei einer Temperatur von 80 °C und einem Permeatdruck von 20 mbar durchgeführt. Als Membran wurde eine Compositemembrane der Universität Köln verwendet.

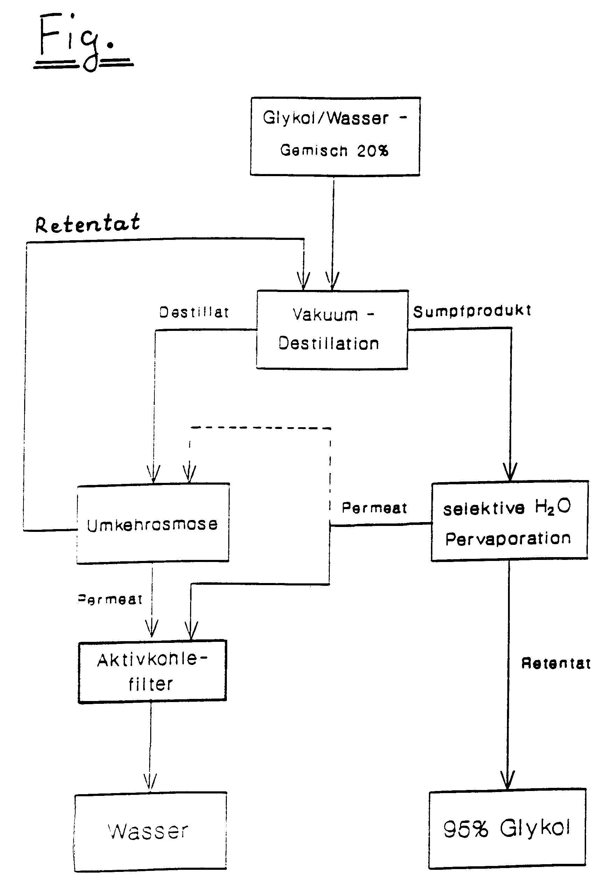

## Patentansprüche

1. Verfahren zur Trennung eines Gemischs aus einer organischen Flüssigkeit und Wasser, wobei solche Flüssigkeiten verwendet werden, die von Wasser mit jedem der Verfahren Destillation, H₂O-selektive Pervaporation und Umkehrosmose getrennt werden können, **gekennzeichnet durch**
- Destillation des Gemischs
- Anwendung einer H₂O-selektiven Pervaporation auf das Sumpfprodukt aus der Destillation
- Anwendung einer Umkehrosmose auf das Destillat
- Rückführung des Retentats aus der Umkehrosmose in den Zufluß der Destillation,
so daß die entmischte organische Flüssigkeit als Retentat aus der H₂O-selektiven Pervaporation vorliegt und das entmische Wasser als Permeat aus der H₂O-selektiven Pervaporation sowie als Permeat aus der Umkehrosmose vorliegt.

2. Verfahren nach Anspruch 1, **dadurch gekennzeichnet,** daß das Permeat aus der H₂O-selektiven Pervaporation in den Zufluß der Umkehrosmose geleitet wird.

3. Verfahren nach einem der vorangehenden Ansprüche, **dadurch gekennzeichnet**, daß die Destillation als Vakuumdestillation durchgeführt wird.

4. Verfahren nach einem der vorangehenden Ansprüche, **dadurch gekennzeichnet**, daß das entmischte Wasser durch ein Aktivkohle-Filter weiter aufbereitet wird.

5. Verfahren nach einem der vorangehenden Ansprüche, **dadurch gekennzeichnet**, daß die organische Flüssigkeit Glykol ist, die in einer Konzentration von 20 bis 30 % im Gemisch enthalten ist.

## Claims

1. A method for separating a mixture comprising an organic fluid and water, such fluids being used which can be separated from water by any one of the processes distillation, H₂O-selective pervaporation, and reverse osmosis, comprising the steps of
- distilling the mixture;
- performing an H₂O-selective pervaporation to bottom product obtained from the distillation step to obtain a residue and permeat;
- applying a reverse osmosis to at least the distillate; and
- feeding the residue obtained from reverse osmosis for performing thereon the distillation step such that regregated organic fluid is present as residue from the H₂O-selective pervaporation, and regregated water is present as the permeate from the H₂O-selective pervaporation step as well as the permeate from the reverse osmosis step.

2. The method according to claim 1, further comprising the step of feeding the permeate from the H₂O-selective pervaporation for performing thereon the reverse osmosis.

3. The method according to any preceding claim, wherin the distillation step is carried out vacuum .distillation

4. The method according to any preceding claim, further comprising the stepof treating the regregated water with an active carbon filter.

5. The method according to any preceding claim, wherin the the organic fluid is glycol contained in the mixture in a concentration of from 20 to 30 %.

## Revendications

1. Procédé de séparation d'un mélange composé d'un liquide organique et d'eau.
Le liquide organique doit pouvoir être séparé de l'eau par distillation, par osmose inversée et par évaporation d'eau à travers une membrane sélective.
Ce procédé est caractérisé par
- la distillation du mélange
- l'évaporation d'eau à travers une membrane sélective pour le résidu de la distillation
- et le retour du rétentat de l'osmose inversée à l'entrée du circuit de distillation
à fin que le liquide organique séparé (sous forme de rétentat) et l'eau (sous forme de perméat) soit le résultat de l'évaporation de l'eau à travers la membrane sélective ainsi que le résultat de l'osmose inversée.

2. Le procédé d'après la revendication 1. est caractérisé par le retour du perméat de l'évaporation d'eau à travers une membrane sélective à l'entrée du circuit de l'osmose inversée.

3. Le procédé d'après les revendications précédentes est caractérisé par le fait que la distillation est une distillation dans le vide.

4. Le procédé d'après les revendications précédentes est caractérisé par le retraitement de l'eau grâce à un filtre au charbon actif.

5. Le procédé d'après les revendications précédentes est caractérisé par le fait que le liquide organique est du glycol avec une concentration de 20 à 30% dans le mélange.
